# EUROPEAN PATENT SPECIFICATION

(11) **EP 1 845 780 B1**
(45) Date of publication and mention of the grant of the patent: **27.07.2011**
(21) Application number: 06719144.5
(22) Date of filing: 20.01.2006
(51) Int. Cl.: A61K 31/4458, A61K 31/445, A01N 43/40, A61P 37/00

(54) **USES OF METHYLPHENIDATE DERIVATIVES**
VERWENDUNG VON METHYLPHENIDAT-DERIVATEN
UTILISATIONS DE DERIVES DE METHYLPHENIDATE

(30) Priority: 20.01.2005 US 645778 P
(43) Date of publication of application: 24.10.2007
(73) Proprietor: Institute for Molecular Medicine, Inc., Englewood CO 80110 (US)
(72) Inventor: BAR-OR, David, Englewood, Colorado 80113 (US); MELAMED, Isaac, Englewood, Colorado 80111 (US)
(74) Representative: Thomas, Simon
(86) International application number: PCT/US2006/002181
(87) International publication number: WO 2006/078984

(56) References cited:
- WO-A-99/16439
- US-B1- 6 658 955
- CHALLMAN T D ET AL: "METHYLPHENIDATA: ITS PHARMACOLOGY AND USES" MAYO CLINIC PROCEEDINGS, MAYO MEDICAL VENTURES, ROCHESTER, MN, vol. 75, no. 7, 1 July 2000 (2000-07-01), pages 711-721, XP008002333 ISSN: 0025-6196
- DAVIES H M L ET AL: "Synthesis of Methylphenidate Analogues and their Binding Affinities at Dopamine and Serotonin Transport Sites" BIOORGANIC & MEDICINAL CHEMISTRY LETTERS, PERGAMON, ELSEVIER SCIENCE, GB, vol. 14, 1 January 2004 (2004-01-01), pages 1799-1802, XP003008608 ISSN: 0960-894X
- BRUERA ET AL.: 'Patient-Controlled Methylphenidate for the Management of Fatigue in Patients With Advanced Cancer: A Preliminary Report' JOURNAL OF ONCOLOGY vol. 21, no. 23, December 2003, pages 4439 - 4443, XP008121370

## Description

### FIELD OF THE INVENTION

The invention relates to uses of methylphenidate derivatives. These uses include inhibiting immune responses and inhibiting inflammation.

### BACKGROUND

Methylphenidate is the treatment of choice for children and adults diagnosed with attention deficit/hyperactivity disorder (ADHD), including its inattentive subtype (formerly known as attention deficit disorder or ADD). Certain derivatives of methylphenidate have also been proposed for the treatment of ADD (see U.S. Patent No. 6,025,502) and for the treatment of other neurological disorders and conditions (see U.S. Patents Nos. 5,859,249, 6,025,502 and 6,486,177 and PCT application WO 99/36403).

Experiments have been performed to examine the effects of methylphenidate on the immune system, and conflicting results have been reported. *See* Auci et al., J. Am. Acad Child Ado/esc. Psychiatry, 36, 1015-1016 (August 1997) and U.S. Patent Application Publication No. 2005/0192290.

### SUMMARY OF THE INVENTION

The invention provides a compound of formula I, or a salt thereof, for use in the treatment of a disease or condition as outlined in the claims, wherein formula I is: 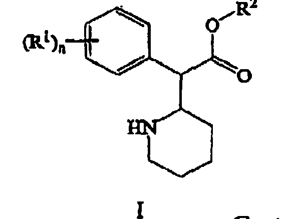 where n is 1 R¹ is to C₆ to C₁₄ aryl or C₆ to C₁₄ aryloxy and R² is methyl.

Specifically,in a first embodiment, the invention provides a compound of formula I for use in inhibiting an immune response in an animal.

In a second embodiment, the invention provides a compound of formula I for use in inhibiting the activation of T-cells in an animal.

In another embodiment, the invention provides a compound of formula I for use in treating a T-cell mediated disease or condition in an animal.

In another embodiment, the invention provides a compound of formula I for use in inhibiting the activation of monocytes in an animal.

In a further embodiment, the invention provides a compound of formula I for use in inhibiting the production and/or release of a proinflammatory cytokine in an animal.

In another embodiment, the invention provides a compound of formula I for use in inhibiting the production and/or release of IL-8 in an animal.

In a further embodiment, the invention provides a compound of formula I for use in inhibiting the production and/or release of IL-13 in an animal.

In another embodiment, the invention provides a compound of formula I for use in inhibiting the production and/or release of interferon gamma (IFNγ) in an animal.

In a further embodiment, the invention provides a compound of formula I for use in of inhibiting the production and/or release of tumor necrosis factor alpha (TNFα) in an animal.

In yet another embodiment, the invention provides a compound of formula I for use in inhibiting inflammation in an animal.

In a further embodiment, the invention provides a compound of formula I for use in treating an inflammatory disease or condition in an animal.

In another embodiment, the invention provides a compound of formula I for use in inhibiting unwanted proliferation of lymphocytes in an animal.

In a further embodiment, the invention provides a compound of formula I for use in inhibiting unwanted adhesion of lymphocytes in an animal.

It is believed that the compounds of formula I are central nervous system stimulants and dopamine uptake inhibitors. Accordingly, in a further embodiment not according to the invention, the invention provides a method of treating an animal suffering from comorbid diseases or conditions, wherein (1) one of the diseases or conditions is a neurological disease or condition which is treatable with a central nervous system stimulant or a dopamine uptake inhibitor and (2) a second disease or condition is an immune or inflammatory disease or condition.

"Inhibit" is used herein to mean to reduce (wholly or partially) or to prevent.

"Mediated" is used herein to mean involving, caused by or exacerbated by.

### BRIEF DESCRIPTION OF THE DRAWINGS

Figures 1A-C are graphs of OD at 530 nm for various additives to peripheral blood lymphocyte (PBL) cultures stimulated with 2 µg/ml, 5 µg/ml and 20 µg/ml phytohemagglutinin (PHA), respectively.

Figure 2 is a graph of OD at 530 nm for various additives to PBL cultures stimulated with 2 µg/ml PHA.

Figure 3 is a graph of concentration of IL-13 for various additives to PBL cultures stimulated with 2 µg/ml PHA.

Figure 4 is a graph of concentration of IFNγ for various additives to PBL cultures stimulated with 2 µg/ml PHA.

Figures 5A-B are graphs of OD at 530 nm for various additives to PBL - cultures stimulated with 2 µg/ml and 5 µg/ml PHA, respectively.

Figure 6 is a graph of concentration of IL-13 for various additives to PBL cultures stimulated with 5 µg/ml PHA.

Figure 7 is a graph of concentration of TNFα for various additives to PBL cultures stimulated with 2 µg/ml PHA.

Figure 8 is a graph of concentration of IL-8 for various additives to PBL cultures stimulated with 2 µg/ml PHA.

Figures 9A-B are graphs of OD for various additives to THP-1 monocyte cultures stimulated with lipopolysaccharide (LPS). In Figure 9B, the top dark gray bar in each instance is c-Jun and the bottom light gray bar is NFκB.

### DETAILED DESCRIPTION OF THE INVENTION

Compounds of formula I are used in the practice of the present invention.

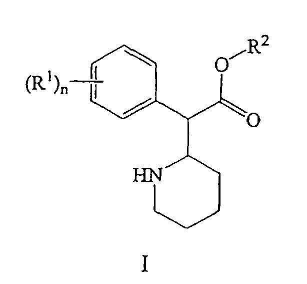

In Formula I, n is 1.

R¹ is C₆ to C₁₄ aryl, or C₆ to C₁₄ aryloxy,
Most preferably R¹ is phenyl.

In formula I,-

R² is -CH₃.

In one specific embodiment, the compound of formula II is particularly useful in the present invention:

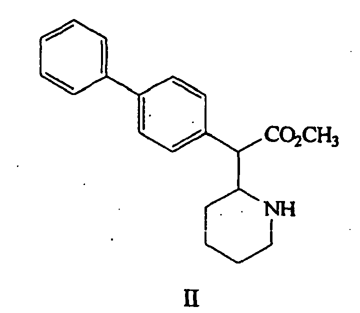

"Acyl" means a moiety of the formula -C(O)-R³, wherein R³ is H, alkyl, cycloalkyl or aryl.

"Amino" means a moiety of the formula -NR⁴R⁵, wherein each of R⁴ and R⁵ is independently H or alkyl, preferably lower alkyl.

"Alkoxy" means a moiety of the formula -OR⁶, wherein R⁶ is an alkyl. An example of an alkoxy group is methoxy (-O-CH₃).

"Alkyl" means a monovalent saturated straight chain or branched hydrocarbon containing 1-8 carbon atoms. Each alkyl may, optionally, be substituted with one or more amino, hydroxyl or sulfhydryl groups.

"Aryl" means a monovalent mono-, bi- or tricyclic aromatic hydrocarbon moiety of 6 to 14 ring carbon atoms. Preferred is phenyl.

"Aryloxy" means a moiety of the formula -OR⁷, wherein R⁷ is an aryl. An example of an aryloxy group is phenoxy.

"Carboxyl" means a moiety of the formula -C(O)-O-R³, wherein R³ is H, an alkyl, cycloalkyl or aryl.

"Cycloalkyl" means a saturated, monovalent mono- or bicyclic hydrocarbon moiety of three to ten ring carbon atoms. Preferably the cycloalkyl contains 4-8 ring carbon atoms. The most preferred cycloalkyl is cyclohexyl.

"Halogen" means chlorine, fluorine, bromine or iodine. Preferred is chlorine or bromine.

"Heteroaryl" means a monovalent monocyclic or bicyclic aromatic moiety of 5 to 12 ring atoms containing one, two, or three ring heteroatoms each of which is independently selected from N, O and S, the remaining ring atoms being C.

"Hydroxyl" means -OH.

"Lower alkyl" means a saturated straight-chain or branched hydrocarbon containing 1-4 carbon atoms.

"Nitro" means -NO₂.

"Sulfhydryl" means -SH.

. "Sulfo" means-SO₃H.

"Prodrug" means any compound which releases an active parent drug according to formula I in vivo when such prodrug is administered to a mammalian subject. Prodrugs of a compound of formula I not according to the invention are prepared by modifying one or more functional group(s) present in the compound of formula I in such a way that the modification(s) may be cleaved in vivo to release the parent compound. Prodrugs include compounds of formula I wherein a hydroxy, amino, or sulfhydryl group in a compound of formula I is bonded to any group that may be cleaved in vivo to generate the free hydroxyl, amino, or sulfhydryl group, respectively. Examples of prodrugs include, but are not limited to, esters (e.g., acetate, formate, and benzoate derivatives), carbumates (e.g., N,N-dimethylaminocarbonyl) of hydroxy functional groups in compounds of formula I, and the like.

"Treating" or "treatment" of a disease or condition includes: (1) preventing the disease or condition, i.e., causing the clinical symptoms of the disease or condition not to develop in a mammal that may be exposed to or predisposed to the disease or condition, but does not yet experience or display symptoms of the disease or condition; (2) inhibiting the disease or condition, i.e., arresting or reducing the development of the disease or condition or its clinical symptoms; or (3) relieving the disease or condition, i.e., causing regression of the disease or condition or its clinical symptoms, including curing the disease or condition.

An "effective amount" means the amount of a compound that, when administered to an animal for treating a disease or condition or for causing an effect is sufficient to do so. The "effective amount" can and will most likely vary depending on the compound, the disease or condition and its severity, or the effect sought to be caused, and the age, weight, etc., of the animal to be treated.

Methods of synthesizing the compounds of formula I useful in the present invention are known in the art. See, *e.g.,* U.S. Patents Nos. 5,859,249 and 6,025,502, PCT application WO 99/36403, Pan et al., Eur. J Pharmacol., 264, 177-182 (1994), Gatley et al., Life Sci., 58, 231-239 (1996), Deutsch et al.. J. Med. Chem., 39, 1201-1209 (1996), Thai et al., J. Med. Chem., 41, 591-601 (1998), and Wayment et al., J. Neurochem., 72:1266-1274 (1999), Krim, Lori, Thesis (Ph.D. in Chemistry) (2001), University Of Pennsylvania, Chemistry Library Reading Room (Call No. QD001 2001.K92), University Microfilms Order No. 3031684, ISBN 0-493-44179-4.

If the compound of the present invention contains one or more chiral centers, the compound can be synthesized enantioselectively or a mixture of enantiomers and/or diastereomers can be prepared and separated. The resolution of the compounds of the present invention, their staging materials and/or the intermediates may be carried out by known procedures, *e.g.*, as described in the four volume compendium Optical Resolution Procedures for Chemical Compounds: Optical Resolution Information Center, Manhattan College, Riverdale, N.Y., and in Enantiomers, Racemates and Resolutions, Jean Jacques, Andre Collet and Samuel H. Wilen; John Wiley & Sons, Inc., New York, 1981, which are incorporated herein in their entirety. Basically, the resolution of the compounds is based on the differences in the physical properties of diastereomers by attachment, either chemically or enzymatically, of an enantiomerically pure moiety, resulting in forms that are separable by fractional crystallization, distillation or chromatography.

The pharmaceutically acceptable salts of the compounds of formula I may also be used in the practice of the invention. Pharmaceutically-acceptable salts include conventional non-toxic salts, such as salts derived from inorganic acids (such as hydrochloric, hydrobromic, sulfuric, phosphoric, nitric, and the like), organic acids (such as acetic, propionic, succinic, glycolic, stearic, lactic, malic, tartaric, citric, glutamic, aspartic, benzoic, salicylic, oxalic, ascorbic acid, and the like) or bases (such as the hydroxide, carbonate or bicarbonate of a pharmaceutically-acceptable metal cation or organic cations derived from N,N-dibenzylethylenediamine, D-glucosanaine, or ethylenediamine). The salts are prepared in a conventional manner, *e.g.*, by reacting the free base form of the compound with an acid.

It is to be understood that the scope of this invention encompasses not only the use of the compounds of formula I themselves, but also the salts thereof. In addition, the present invention contemplates the use of the isomers of the compounds of formula I, and of the salts thereof, including pure isomers and various mixtures of isomers.

Compounds of formula I, or pharmaceutically-acceptable salts thereof , can be used as immunosuppressants. These compounds inhibit a wide variety of immune responses.

For instance, a compound of formula I can be used to inhibit the activation of T-cells and to treat T-cell mediated diseases. T-cell mediated diseases treatable according to the invention include graft rejection, graft versus host disease, unwanted delayed-type hypersensitivity reactions (such as delayed-type allergic reactions), T-cell mediated pulmonary diseases, autoimmune diseases and T-cell mediated inflammation. T-cell mediated pulmonary diseases include sarcoidosis, hypersensitivity pneumonitis, acute interstitial pneumonitis, alveolitis, pulmonary fibrosis, idiopathic pulmonary fibrosis and other diseases characterized by inflammatory lung damage. Autoimmune diseases include multiple sclerosis, neuritis, polymyositis, psoriasis, vitiligo, Sjogren's syndrome, rheumatoid arthritis, Type 1 diabetes, autoimmune pancreatitis, inflammatory bowel diseases (*e.g.*, Crohn's disease and ulcerative colitis), celiac disease, glomerulonephritis, scleroderma, sarcoidosis, autoimmune thyroid diseases (*e.g.*, Hashimoto's thyroiditis and Graves disease), myasthenia gravis, Addison's disease, autoimmune uveoretinitis, pemphigus vulgaris, primary biliary cirrhosis, pernicious anemia, and systemic lupus erythematosia.

A compound of formula 1 can also be used to inhibit the production, release, or both, of cytokines, including interleukin-8 (IL-8), IL-13, interferon gamma (IFNγ) and tumour necrosis factor alpha (TNFa). IL-8 is a pro-inflammatory cytokines and a potent chemoattractant and activator of neutrophils. It has also been reported to be a chemoattractant and activator of T-lymphocytes and eosinophils. IL-8 is produced by immune cells (including lymphocytes, neutrophils, monocytes and macrophages), fibroblasts and epithelial cells. IL-13 is made by activated T_{H}2 cells, and IL-13's primary targets are B-cells and monocytes. IL-13 stimulates humoral immune responses, and it has been implicated in the pathogenesis of asthma. IFNγ and TNFα are both proinflammatory cytokines made by activated T-cells and other cells. TNFα rapidly activates the transcription factors NFκB and AP-1, causes endothelial cells to express adhesion molecules, and may play a role in the recruitment of immune cells to the sites of inflammation. IFNγ can activate neutrophils, endothelial cells and macrophages, as well as cause an increase in MHC molecule expression. IFNγ drives the cell-mediated immune response.

A compound of formula I, or a pharmaceutically-acceptable salt thereof , can be used to inhibit inflammation and to treat inflammatory diseases and conditions. An inflammatory disease or condition is a disease or condition which involves, or is caused or exacerbated by, inflammation. Specific inflammatory diseases and conditions treatable with a compound of the present invention include acute respiratory distress syndrome, allergies, arthritis, asthma, autism, autoimmune diseases (*e.g,* multiple sclerosis and systemic lupus erythematosis), bronchitis, cancer, Crohn's disease, cystic fibrosis, emphysema, endocarditis, gastritis, infections (bacterial, viral, yeast, fungal and parasitic), inflammatory bowel disease, inflammatory skin disorders ischemia reperfusion, multiple organ dysfunction syndrome, multiple organ failure, nephritis, neurodegenerative diseases (*e.g.*, Alzheimer's disease, amyotrophic lateral sclerosis, Huntington's chorea, Parkinson's disease and dementia), pancreatitis, psoriasis, respiratory viral infections, sepsis, shock, systemic inflammatory response syndrome, trauma, ulcerative colitis and other inflammatory diseases, conditions and disorders. The compounds of the present invention, including their pharmaceutically-acceptable salts, will be especially useful for treating inflammatory neurological diseases and conditions, such as autism, Alzheimer's disease, multiple sclerosis, Huntington's chorea, Parkinson's disease, amyotrophic lateral sclerosis, senile dementia and dementia associated with HTV infections.

A compound of formula I, or a pharmaceutically-acceptable salt thereof , can be used to inhibit unwanted proliferation of lymphocytes. Unwanted proliferation of lymphocytes occurs in, *e.g.*, certain types of cancer (*e.g.*, thymomas, lymphomas and leukemias) and inflammation.

A compound of formula I, or pharmaceutically-aceptable salt thereof , can be used to inhibit unwanted adhesion of lymphocytes. Unwanted adhesion of lymphocytes is involved in, *e.g.*, inflammation, inflammatory diseases and conditions, and immune responses.

In addition to the activities described above, the compounds of the present invention are believed to be central nervous system stimulants and dopamine uptake inhibitors. Accordingly, they will be especially useful for treating an animal suffering from comorbid diseases or conditions not according to the invention wherein (1) one of the diseases or conditions is a neurological disease or condition treatable with a central nervous system stimulant or a dopamine uptake inhibitor and (2) a second disease or condition is an immune or inflammatory disease or condition. Neurological diseases and conditions treatable with a central nervous system stimulant include ADHD, depression, dysphoria, narcolepsy, fatigue (*e.g.*, due to chemotherapy or treatment with narcotic analgesics), hypersomnia, cognitive disorders, compulsive shopping disorder. Neurological diseases and conditions treatable with a dopamine uptake inhibitor include cocaine abuse or addiction and Parkinson's disease (alone or in combination with dopa, levodopa or another dopamine precursor). Immune and inflammatory diseases and conditions include those described above. In a particularly preferred embodiment, the compounds of the present invention and their pharmaceutically-acceptable salts will be used to treat comorbid ADHD and allergies,

To treat an animal in need of treatment, a compound of formula I, or a pharmaceutically-acceptable salt thereof, is administered to the animal. Preferably, the animal is a mammal, such as a rabbit, goat, dog, cat, horse or human. Most preferably, the animal is a human.

Effective dosage forms, modes of administration and dosage amounts for the compounds of the invention may be determined empirically, and making such determinations is within the skill of the art. It is understood by those skilled in the art that the dosage amount will vary with the particular compound employed, the disease or condition to be treated, the severity of the disease or condition, the route(s) of administration, the rate of excretion of the compound, the duration of the treatment, the identify of any other active ingredient(s) being administered to the animal, the age, size and species of the animal, and like factors known in the medical and veterinary arts. In general, a suitable daily dose of a compound of the present invention will be that amount of the compound which is the lowest dose effective to produce a therapeutic effect. However, the daily dosage will be determined by an attending physician or veterinarian within the scope of sound medical judgment. If desired, the effective daily dose may be administered as two, three, four, five, six or more sub-doses, administered separately at appropriate intervals throughout the day. Administration of the compound should be continued until an acceptable response is achieved.

The compounds useful in the present invention (*i.e.*, the compounds of formula I and the pharmaceutically-acceptable salts) may be administered to an animal patient for therapy by any suitable route of administration, including orally, nasally, rectally, vaginally, parenterally (*e.g.*, intravenously, intraspinally, intraperitoncally, subcutaneously, or intramuscularly), intracisternally, transdermally, intracranially, intracerebrally, and topically (including buccally and sublingually). The preferred route of administration is orally.

While it is possible for a compound useful in the present invention to be administered alone, it is preferable to administer the compound as a pharmaceutical formulation (composition). The pharmaceutical compositions useful in the invention comprise one or more compounds of formula I, or pharmaceutically-acceptable salts as active ingredient(s) in admixture with one or more pharmaceutically-acceptable carriers and, optionally, with one or more other compounds, active ingredient(s) or other materials. Each carrier must be "acceptable" in the sense of being compatible with the other ingredients of the formulation and not injurious to the animal. Pharmaceutically-acceptable carriers are well known in the art. Regardless of the route of administration selected, the compounds of the present invention are formulated into pharmaceutically acceptable dosage forms by conventional methods known to those of skill in the art. See, *e.g.*, *Remington's Pharmaceutical Sciences.*

Formulations of the invention suitable for oral administration may be in the form of capsules, cachets, pills, tablets, powders, granules or as a solution or a suspension in an aqueous or non-aqueous liquid, or an oil-in-water or water-in-oil liquid emulsions, or as an elixir or syrup, or as pastilles (using an inert base, such as gelatin and glycerin, or sucrose and acacia), and the like, each containing a predetermined amount of a compound or compounds useful in the present invention as an active ingredient. A compound or compounds useful in the present invention may also be administered as bolus, electuary or paste.

In solid dosage forms for oral administration (capsules, tablets, pills, dragees, powders, granules and the like), the active ingredient(s) is (are) mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monosterate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may be employed as fillers in soft and hard-filled gelatin capsules using such excipients as lactose or milk sugars, as well as high molecular weight polyethylene glycols and the like.

A tablet may be made by compression or molding optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter. These compositions may also optionally contain opacifying agents and may be of a composition that they release the active ingredient only, or preferentially, in a certain portion of the gastrointestinal tract, optionally, in a delayed manner. Examples of embedding compositions which can be used include polymeric substances and waxes. The active ingredient can also be in microencapsulated form.

Liquid dosage forms for oral administration of the compounds of the invention include pharmaceutically-acceptable emulsions, microemulsions, solutions, suspensions, syrups and elixirs. In addition to the active ingredient(s), the liquid dosage forms may contain inert diluents commonly used in the art, such as, for example, water or other solvents, solubilizing agents and emulsifiers, such as ethyl alcohol, isopropyl alcohol; ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compound(s), may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or salicylate, and which is solid at room temperature, but liquid at body temperature and, therefore, will melt in the rectum or vaginal cavity and release the active compound. Formulations of the present invention which are suitable for vaginal administration also include pessaries, tampons, creams, gels, pastes, foams or spray formulations containing such carriers as are known in the art to be appropriate.

Dosage forms for the topical or transdermal administration of compounds useful in this invention include powders, sprays, ointments, pastes, creams, lotions, gels, solutions, patches, drops and inhalants. The active ingredient(s) may be mixed under sterile conditions with a pharmaceutically-acceptable carrier, and with any buffers, or propellants which may be required.

The ointments, pastes, creams and gels may contain, in addition to the active ingredient(s), excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the active ingredient(s), excipients such as lactose, talc, silicic acid, aluminum hydroxide, calcium silicates and polyamide powder or mixtures of these substances. Sprays can additionally contain customary propellants such as chlorofluorohydrocarbons and volatile unsubstituted hydrocarbons, such as butane and propane.

Transdermal patches have the added advantage of providing controlled delivery of compounds of the invention to the body. Such dosage forms can be made by dissolving, dispersing or otherwise incorporating one or more compounds of the invention in a proper medium, such as an elastomeric matrix material. Absorption enhancers can also be used to increase the flux of the compound across the skin. The rate of such flux can be controlled by either providing a rate-controlling membrane or dispersing the compound in a polymer matrix or gel.

Pharmaceutical formulations include those suitable for administration by inhalation or insufflation or for nasal or intraocular administration. For administration to the upper (nasal) or lower respiratory tract by inhalation, the compounds of the invention are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlolotetrafluoroethane, carbon dioxide, or other suitable gas. In the case of a pressurized aerosol, the dosage unit may be determined by providing a valve to deliver a metered amount

Alternatively, for administration by inhalation or insufflation, the composition may take the form of a dry powder, for example, a powder mix of one or more compounds of the invention and a suitable powder base, such as lactose or starch. The powder composition may be presented in unit dosage form in, for example, capsules or cartridges, or, *e.g*., gelatin or blister packs from which the powder may be administered with the aid of an inhalator, insufflator or a metered-dose inhaler.

For intranasal administration, compounds useful in the invention may be administered by means of nose drops or a liquid spray, such as by means of a plastic bottle atomizer or metered-dose inhaler. Typical of atomizers are the Mistometer (Wintrop) and Medihaler (Riker).

Drops, such as eye drops or nose drops, may be formulated with an aqueous or nonaqueous base also comprising one or more dispersing agents, solubilizing agents or suspending agents. Liquid sprays are conveniently delivered from pressurized packs. Drops can be delivered by means of a simple eye dropper-capped bottle or by means of a plastic bottle adapted to deliver liquid contents dropwise by means of a specially shaped closure.

Pharmaceutical compositions suitable for parenteral administrations comprise one or more compounds useful in the invention in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as wetting agents, emulsifying agents and dispersing agents. It may also be desirable to include isotonic agents, such as sugars, sodium chloride, and the like in the compositions. In addition, prolonged absorption of the injectable pharmaceutical form may be brought about by the inclusion of agents which delay absorption such as aluminum monosterate and gelatin.

In some cases, in order to prolong the effect of the active ingredient(s), it is desirable to slow the absorption of the active ingredient(s) from subcutaneous or intramuscular injection. This may be accomplished by the use of a liquid suspension of crystalline or amorphous material having poor water solubility. The rate of absorption of the active ingredient(s) then depends upon its rate of dissolution which, in turn, may depend upon crystal size and crystalline form. Alternatively, delayed absorption of a parenterally-administered active ingredient(s) is accomplished by dissolving or suspending the active ingredient(s) in an oil vehicle.

Injectable depot forms are made by forming microencapsule matrices of the active ingredient(s) in biodegradable polymers such as polylactide-polyglycolide. Depending on the ratio of active ingredient(s) to polymer, and the nature of the particular polymer employed, the rate of release of the active ingredient(s) can be controlled. Examples of other biodegradable polymers include poly(orthoesters) and poly(anhydrides). Depot injectable formulations are also prepared by entrapping the active ingredient(s) in liposomes or microemulsions which are compatible with body tissue. The injectable materials can be sterilized for example, by filtration through a bacterial-retaining filter.

The formulations may be presented in unit-dose or multi-dose sealed containers, for example, ampules and vials, and may be stored in a lyophilized condition requiring only the addition of the sterile liquid carrier, for example water for injection, immediately prior to use. Extemporaneous injection solutions and suspensions may be prepared from sterile powders, granules and tablets of the type described above.

Additional objects, advantages, and novel features of this invention will become apparent to those skilled in the art upon examination of the following examples thereof, which are not intended to be limiting.

### EXAMPLES

### EXAMPLE 1

Whole blood was drawn from GR283, a human volunteer with known allergies, into a glass vacutainer tube containing no anticoagulant. This blood was allowed to clot, and the serum was removed by centrifugation and then heat inactivated by placing it in a water bath at 56°C for 30 minutes. Whole blood from GR283 was also drawn into a glass vacutainer tube containing heparin and used for peripheral blood lymphocytes (PBL) isolation as follows. Whole blood was layered over room temperature Histopaque 1077 solution and centrifuged at 2000 rpm for 15 minutes at room temperature. Cells at the plasma-Histopaque interface were then removed and washed with culture medium (IMDM medium with 10% heat-inactivated GR283 serum plus 1% penicillin/streptomycin) at 37 °C.

The compound of formula II (see above) and methylphenidate (both obtained from Dr. Jeffrey D. Winkler, University of Pennsylvania, Philadelphia, Pennsylvania) in culture medium were added to wells of a 96-well plate to give final concentrations of 5 µg/ml, 15 µg/ml and 50 µg/ml of the compound of formula II and of methylphenidate. Sterile 18 MΩ water, the solvent for the compound of formula II, and dexamethasone (obtained from Sigma) (final concentration of 10 µg/ml in water) were used as controls. Then, GR283's PBL in culture medium were added to the wells to give a final concentration of 150,000 cells per well, and the plates were incubated at 37°C, 5% CO₂ for 24 hours. After-this incubation, phytohemagglutinin (PHA) in culture medium was added to give final concentrations of 2 µg/ml, 5 µg/ml or 20 µg/ml, final total volume of 200 µl/well, and the cells were incubated for an additional 72 hours at 37°C, 5% CO₂. All cultures were performed in triplicate.

At the end of this incubation, cell clumping was examined by photographing representative wells with a digital camera mounted to an inverted microscope. The compound of formula II reduced the amount of cell clumping induced by 5 µg/ml PHA in a dose-dependent manner. The compound of formula II attenuated cell clumping, presumably, as a result of decreased expression of cellular adhesion molecules on the surfaces of the cells.

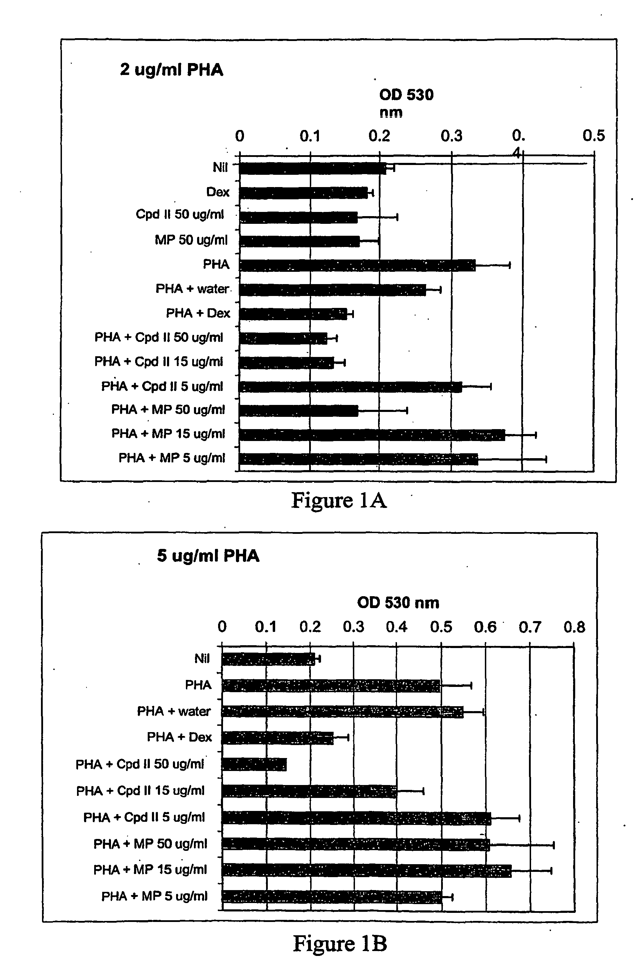

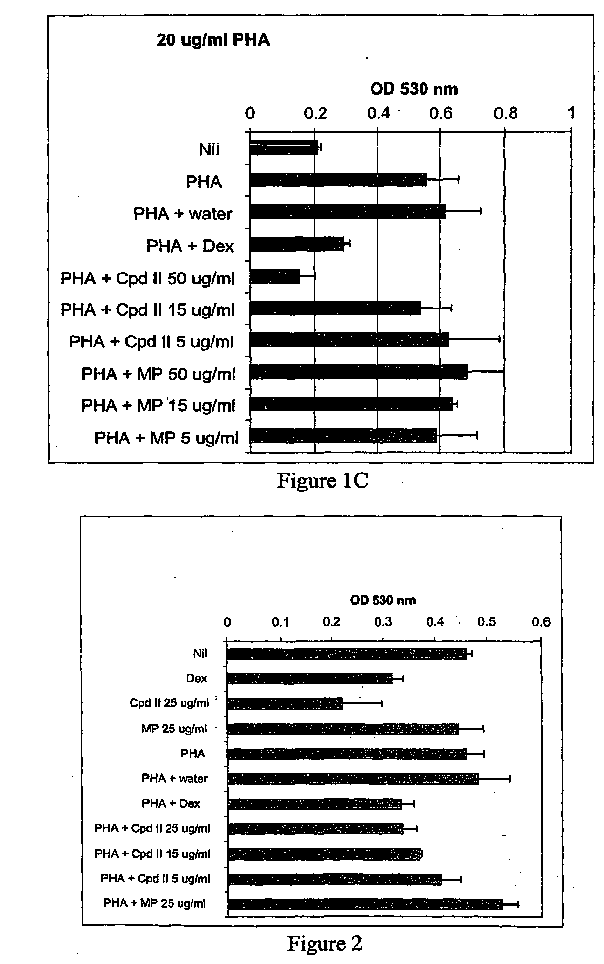

Cell proliferation was assayed by adding 20 µl of Promega cell titer solution to each well and incubating the plate for an additional 4 hours. Promega cell titer solution is a solution containing a tetrazolium dye that is reduced by living cells to a formazan dye, and this reduction is proportional to the number of living cells present in the well. After the 4-hour incubation, the optical density (OD) at 530 nm of each well was measured. The OD at 530 nm for blank wells containing no cells was subtracted from the OD of the experimental wells. The results of the proliferation assays are presented in Figures 1A-C. As can be seen from Figures 1A-C, the compound of formula II (Cpd. II) and dexamethasone (Dex) significantly inhibited the proliferation of PBL stimulated with PHA in a dose-dependent manner. Methylphenidate (MP) showed a significant effect at its highest dose and the lowest PHA dose. Otherwise, methyphenidate did not significantly reduce the proliferation of the PHA-stimulated PBL.

### EXAMPLE 2

Whole blood was drawn from GR467, a human volunteer with known allergies, and processed as described in Example 1 to give heat-inactivated serum and PBL. The compound of formula II and methylphenidate in culture medium (made using heat-inactivated GR467 serum) were added to wells of a 96-well plate to give final concentrations of 5 µg/ml, 15 µg/ml and 25 µg/ml of the compound of formula II and 15 µg/ml methylphendiate. Water and dexamethasone (final concentration of 10 µM) were used as controls. Then, GR467's PBL in culture medium were added to the wells to give a final concentration of 150,000 cells per well, and the plates were incubated at 37°C, 5% CO₂ for 24 hours. After this incubation, PHA was added to give a final concentration of 2 µg/ml, final total volume of 200 µl/well, and the cells were incubated for an additional 72 hours at 37°C, 5% CO₂. All cultures were performed in triplicate.

At the end of this incubation, cell proliferation was determined as described in Example 1. The results are presented in Figure 2. As can be seen from Figure 2, the compound of formula II (Cpd II) and dexamethasone (Dex) significantly inhibited the proliferation of PBL, both unstimulated and stimulated with PHA, whereas methylphenidate did not.

The release of cytokines by the PBL was also measured by culturing the PBL in 1 ml tubes, at 1.3 x 10⁶ cells per ml, with 15 µg/ml of the compound of formula II, 15 µg/ml methylphenidate or 10 µM dexamethasone at 37°C, 5% CO₂ for 24 hours. After this incubation, PHA was added to give a final concentration of 2 µg/ml, and the cells were incubated for an additional 96 hours at 37°C, 5% CO₂. All cultures were performed in triplicate. Cells were then removed by centrifugation at 1000 rpm for 10 minutes, and the culture medium collected.

Release of IL-13 and interferon gamma (IFNγ) into the culture medium was measured by ELISA. To perform the ELISA, matched pairs of antibodies against human IL-13 and IFNγ were purchased from Pierce Biotechnology and Biosource, respectively. ELISA strip well plates were coated with 10 µg/ml of antibody (in phosphate-buffered saline (PBS)) to IL-13 and 4 µg/ml of antibody to IFNγ (in PBS) overnight at room temperature. The plates were then blocked using a 4% BSA solution in PBS for one hour, followed by the addition of 50 µl of experimental culture medium per well in duplicate. The plates were incubated at room temperature for one hour and then washed using 50 mM Tris pH 8.0 with 0.1 % Tween 20. Then, solutions of 400 ng/ml biotinylated second antibody to IL-13 and 500 ng/ml biotinylated second antibody to IFNγ were made in blocking buffer, and 100 µl were added per well. The plates were incubated for 1 hour and washed again. A 1:8000 dilution of Strepavidin HRP (Pierce Biotechnology) conjugate was made in blocking buffer, and 100 µl were added to the wells and incubation continued for 30 minutes. A final wash step was performed, after which 100µl Pierce Biotechnology TMB substrate were added to each well. Color was developed for 30 minutes and stopped by adding 100 µl 0.18 N H₂SO₄. OD was determined using microplate reader with a 450 nM filter.

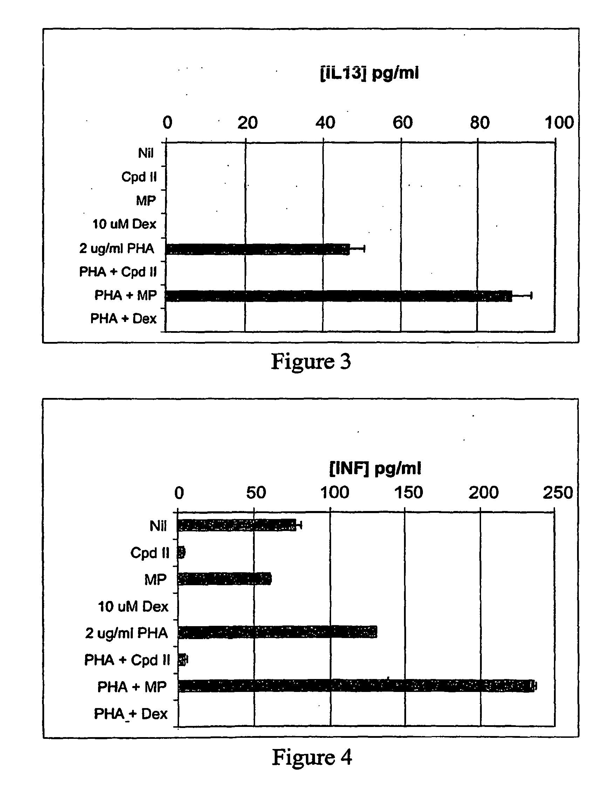

The results for IL-13 are shown in Figure 3. As can be seen, the compound of formula II (Cpd. II) and dexamethasone (Dex) significantly inhibited IL-13 release induced by PHA. Methylphenidate (MP) did not inhibit the release of IL-13. Indeed, methylphenidate increased the release of IL-13 by the PHA-stimulated cells.

The results for IFNγ are shown in Figure 4. As can be seen, the compound of formula II (Cpd. II) and dexamethasone (Dex) significantly inhibited IFNγ release in both unstimulated cells and in cells stimulated with PHA. Methylphenidate (MP) had some effect on the release of IFNγ by unstimulated cells, but did not significantly suppress the release of IFNγ from cells stimulated with PHA. Indeed, methylphenidate increased the release of IFNγ by the PHA-stimulated cells.

### EXAMPLE 3

Whole blood was drawn from GR191, a normal human volunteer, and processed as described in Example 1 to give heat-inactivated serum and PBL. The compound of formula II and methylphenidate in culture medium (made using heat-inactivated GR191 serum) were added to wells of a 96-well plate to give final concentrations of 5 µg/ml, 15 µg/ml, 25 µg/ml and 50 µg/ml of the compound of formula II and 50 µg/ml methylphendiate. Water, mouse nerve growth factor (Upstate Biotechnology, Inc) (NGF) (final concentration of 250 ng/ml) and dexamethasone (final concentration of 10 µM) were used as controls. Then, GR191's PBL in culture medium were added to the wells to give a final concentration of 150,000 cells per well, and the plates were incubated at 37°C, 5% CO₂ for 24 hours. After this incubation, PHA was added to give final concentrations of 2 µg/ml and 5 µg/ml, final total volume of 200 µl/well, and the cells were incubated for an additional 72 hours at 37°C, 5% CO₂. All cultures were performed in triplicate.

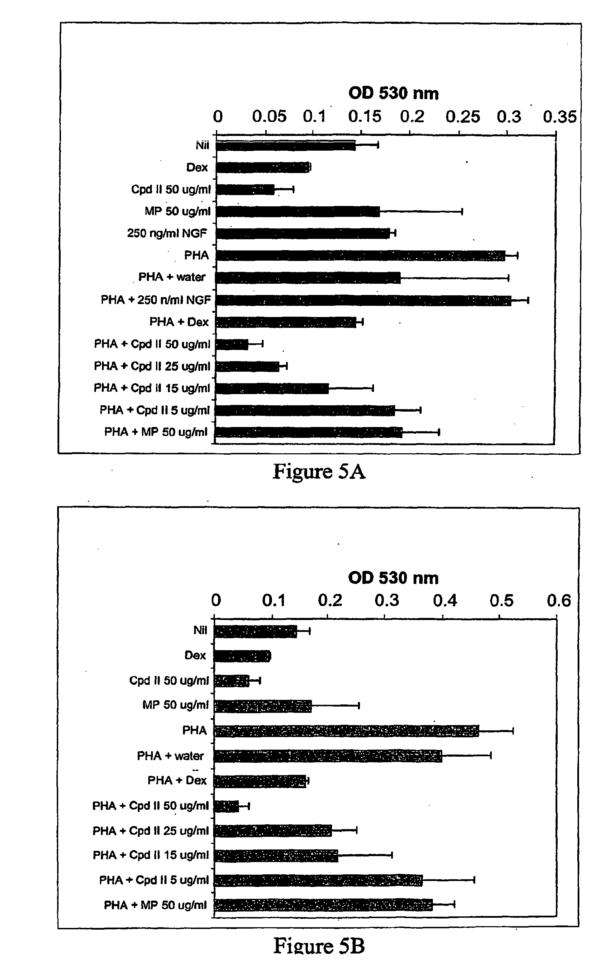

At the end of this incubation, cell proliferation was determined as described in Example 1. The results are presented in Figures 5A-B. As can be seen from Figures 5A-B, the compound of formula II (Cpd. II) and dexamethasone (Dex) significantly inhibited the proliferation of PBL, both unstimulated and stimulated with PHA, whereas methylphenidate (MP) did not.

The release of cytokines by the PBL was also measured by culturing the PBL in 1 ml tubes, at 1 x 10⁶ cells per ml, with 15 µg/ml and 50 µg/ml of the compound of formula II or 10 µM dexamethasone at 37°C, 5% CO₂ for 24 hours. After this incubation, PHA was added to give a final concentration of 5 µg/ml, and the cells were incubated for an additional 72 hours at 37°C, 5% CO₂. All cultures were performed in triplicate. Cells were then removed by centrifugation at 1000 rpm for 10 minutes.

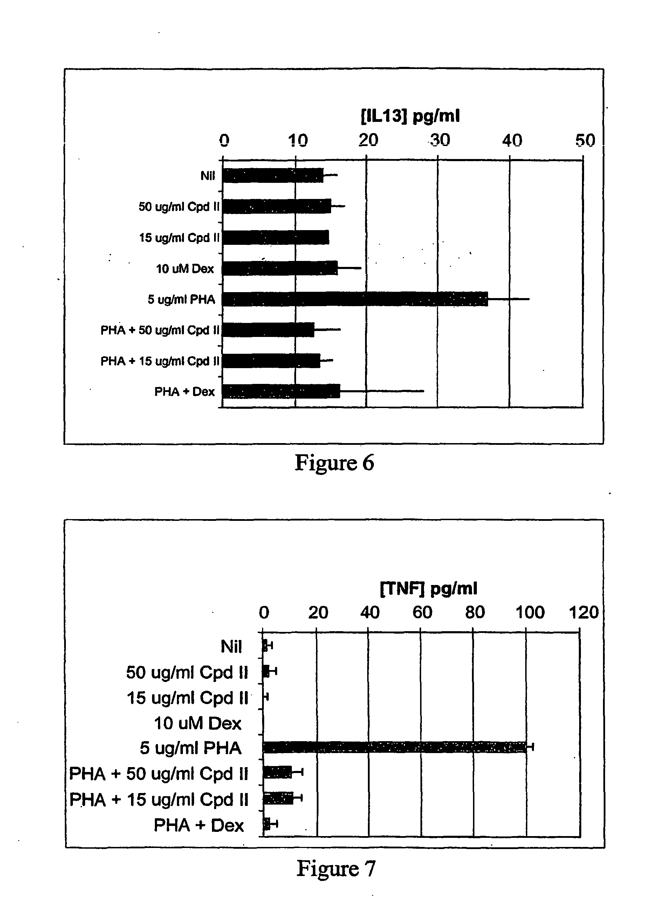

The supernatants were collected, and the concentrations of IL-13 and tumor necrosis factor alpha (TNFα) in the supernatants were measured by ELISA. The IL-13 ELISA was performed as described in Example 2. The results are presented in Figure 6. As can be seen in Figure 6, the compound of formula II (Cpd. II) and dexamethasone (Dex) significantly inhibited the release of IL-13 from the PHA-stimulated PBL.

The TNFα ELISA was performed as described in Example 2 using matched pair antibodies from Pierce Endogen (2 µg/ml for the coating antibody and 250 ng/ml for the second antibody). The results are presented in Figure 7. As can be seen in Figure 7, the compound of formula II (Cpd. II) and dexamethasone (Dex) significantly inhibited the release of TNFα from PHA-stimulated PBL.

The cells were further analyzed by flow cytometry. Annexin was used to determine populations of dead or dying cells. Anti-CD69 antibody was used to establish the level of cellular activation. Antibody to T-cell receptor αβ (TCR) was also used. Recombinant Annexin 5 (PE and FITC conjugates) and the antibodies were all purchase from Caltag (Burlingham, CA) and used following the manufacturer's recommendations. The following results were observed.

### Cell Death:

Annexin staining of TCR-positive cells increased from 7.3% (background) to 45% and 23% with 50 µg/ml and 15 µg/ml of the compound of formula II, respectively, signifying an increase in cell death in the T-cell population. Stimulation with PHA at 5 µg/ml increased the annexin staining of TCR-positive cells to 67%. This indicates that PHA can also induce cell death in the T-cell population. Cell death decreased slightly as a result of treatment with PHA plus 15 µg/ml of the compound of formula II (62% of the TCR-positive cells stained for annexin with PHA and IMM 0001 versus 67% with PHA alone). PHA plus 50 µg/ml of the compound of formula II caused 87% cell death in the TCR-positive subset of cells as seen by annexin staining. These results show that the higher 50 µg/ml concentration of the compound of formula II caused significant death of T-cells, whereas the lower 15 µg/ml concentration did not. Dexamethasone rescued the PHA-induced increase in annexin staining of TCR-positive cells (decreased from 84% to 48%), demonstrating that the control - compound is working properly.

### Activation Of T-Cells:

CD69 + TCR staining (activated T cells) was not detected in any of the controls (nil, compound of formula II alone and dexamethasone alone). PHA increased CD69 + TCR staining to 84%. Only PHA caused T-cell activation as detectable by increased CD 69 staining. CD69 + TCR staining of PHA-stimulated cells dropped from 84% to 54% with 50 µg/ml of the compound of formula II and to 64% with 15 µg/ml of the compound of formula II. Dexamethasone was less effective than the compound of formula II at reducing the CD69 + TCR staining of PHA-stimulated cells. Thus, the compound of formula II is more effective at decreasing T-cell activation than dexamethasone, a potent anti-inflammatory.

### EXAMPLE 4

Whole blood was drawn from GR-192, a normal human volunteer, and processed as described in Example 1 to give heat-inactivated serum and PBL. Then, GR-192's PBL were cultured in 1 ml tubes, at 1.3 x 10⁶ cells per ml, with 15 µg/ml of the compound of formula II (in culture medium made using 10% heat-inactivated GR-192 serum) or 10 µM dexamethasone, at 37°C, 5% CO₂ for 24 hours. After this incubation, PHA was added to give a final concentration of 2 µg/ml, and the cells were incubated for an additional 96 hours at 37°C, 5% CO₂. All cultures were performed in triplicate. Cells were then removed by centrifugation at 1000 rpm for 10 minutes, and the culture medium collected.

Release of IL-8 into the culture medium was measured by ELISA. To perform the ELISA, matched pairs of antibodies against human IL-8 were purchased from Pierce Biotechnology and Biosource, respectively. ELISA strip well plates were coated with 2 µg/ml of antibody to IL-8 (in phosphate-buffered saline (PBS)) overnight at room temperature. The plates were then blocked using a 4% BSA solution in PBS for one hour, followed by the addition of 50 µl of experimental culture medium per well in duplicate. The plates were incubated at room temperature for one hour and then washed using 50 mM Tris pH 8.0 with 0.1 % Tween 20. Then, solutions of 100 ng/ml biotinylated second antibody to IL-8 were made in blocking buffer, and 100 µl were added per well. The plates were incubated for 1 hour and washed again. A 1:8000 dilution of Strepavidin HRP (Pierce Biotechnology) conjugate was made in blocking buffer, and 100 µl were added to the wells and incubation continued for 30 minutes. A final wash step was performed, after which 100 µl Pierce Biotechnology TMB substrate were added to each well. Color was developed for 30 minutes and stopped by adding 100 µl 0.18 N H₂SO₄. OD was determined using microplate reader with a 450 nm filter.

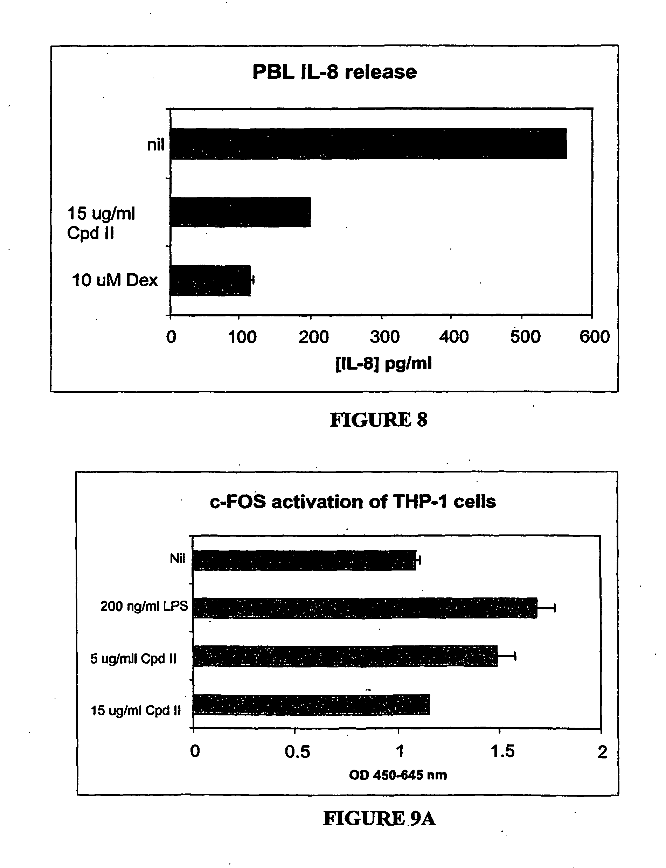

The results are shown in Figure 8. As can be seen, the compound of formula II (Cpd. II) and dexamethasone (Dex) significantly inhibited IL-8 release induced by PHA.

A CD4-positive human T-lymphocyte cell line (TRiPS), which was isolated from an influenza-immunized donor and is specific for hemagglutinin peptide 307-319, was stimulated for passage using approximately 4x10⁵ cells on day 18-20 after a previous stimulation. Cells were washed once in cold Iscove's Modified Dulbecco Minimal Essential Medium (IMDM, Sigma) plus 10% fetal calf serum (FCS; American Type Culture Collection (ATCC)) and resuspended in 1.0 ml cold IMDM medium containing a 1:500 dilution of anti-CD3 monoclonal antibody OKT3 (prepared from mouse ascites fluid). Cells were incubated with antibody for 30 minutes on ice, then washed with cold medium without FCS and combined with approximately 2x10⁶ 4000R-irradiated normal human donor peripheral blood leukocytes (PBL), as feeder cells, in medium plus 50 U/ml human IL-2 (Xenometrix). Cultures were expanded by the addition of fresh IMDM medium with FCS plus IL-2 on day 3. Day of culture is measured from the day of stimulation with OKT3. Cells can be used for experiments starting on day 7 (at maximum proliferation), typically on day 14 (most sensitive to re-stimulation) and up until day 21 (resting cells approaching senescence).

Activation experiments were performed by withdrawing an aliquot of cells and washing twice with warmed (37°C) IMDM. For each specific assay, 2x10⁵ viable cells were pre-incubated in a total volume of 0.9 ml warmed IMDM medium containing 15 µg/ml of the compound of formula II or 10 µM dexamethasone for 15 minutes at 37°C. An aliquot of 2x10⁵ CD3/CD28 Dynabeads (Dynal), as activating stimulus, in 0.1 ml warmed IMDM was then added, and the cultures incubated 24 hours at 37°C. Supernatants of the cell cultures were harvested after pelleting the cells by centrifugation.

Cytokine content was assayed by specific IL-8 ELISA as described above. It was found that the compound of formula II had no effect on IL-8 production by the TRiPS cell line.

### EXAMPLE 5

THP-1 is a monocyte cell line obtained from American Type Culture Collection (ATCC) (catal og no. TIB-202). THP-1 cells were placed in medium (RPMI containing 10 % fetal calf serum (FCS) (obtained from ATCC) and 8 ng/ml monothioglycerol (obtained from Sigma)) at a concentration of 250,000 cells per ml and incubated with 15 µg/ml of compound of formula II or 10 µM dexamethasone for one hour at 37°C and 5% CO₂. After 1 hour, lipopolysaccharide (LPS) (obtained from Sigma) was added to the cultures to give a final concentration of 200 ng/ml, and the cells were then incubated for an additional 4 hours or for an additional 24 hours. After the incubation, the cells were centrifuged, and the supernatants were collected. The concentrations of IL-8 and TNFα in the supernatants were determined by ELISA.

The concentrations of IL-8 in the supernatants were determined by ELISA performed as described in Example 4. The results are presented in Table 1 below. As can be seen in Table 1, the compound of formula II (Cpd. II) and dexamethasone (Dex) significantly inhibited the release of IL-8 from the LPS-stimulated monocytes.

The TNFα ELISA was performed as described in Example 2. The results are presented in Table 2 below. As can be seen in Table 2, the compound of formula II (Cpd. II) and dexamethasone (Dex) significantly inhibited the release of TNFα from the LPS-stimulated monocytes.

**TABLE 1**

| Sample | Time Of Incubation | Mean IL-8 Concentration (pg/ml) | % Inhibition |
|---|---|---|---|
| Control (no additives) | 4 hours | 75.96 ± 12.73 | N/A |
| LPS | 4 hours | 2844.60 ± 180.55 | N/A |
| LPS+Cpd II | 4 hours | 2185.00 ± 78.30 | 23% |
| LPS + Dex | 4 hours | 2102.18 ± 52.20 | 26% |
| | | | |
| Control (no additives) | 24 hours | 46.09 ± 22.42 | N/A |
| LPS | 24 hours | 6653.20 ± 193.18 | N/A |
| LPS + Cpd II | 24 hours | 4490.20 ± 264.46 | 33% |
| LPS + Dex | 24 hours | 2300.00 ± 283.41 | 66% |

**TABLE2**

| Sample | Time Of Incubation | Mean TNFα Concentration (pg/ml) | % Inhibition |
|---|---|---|---|
| Control (no additives) | 24 hours | 1.415 ± 1.464 | N/A |
| LPS | 24 hours | 138.655 ± 0.601 | N/A |
| LPS + Cpd II | 24 hours | 65.370 ± 0.891 | 53% |
| LPS + Dex | 24 hours | 94.759 ± 8.755 | 32% |

### EXAMPLE 6

Cells of the MC/9 murine fibroblast cell line (obtained from ATCC, catalog no. CRL-8305) were placed into the wells of a 96-well tissue culture plate at 25,000 cells/well. The culture medium was Delbecco's Modified Eagle's Medium (DMEM) (obtained from Cambrex) containing 10% fetal calf serum (FCS) (obtained from ATCC). Nil control wells contained no additives. The remaining wells contained either 25 ng/ml murine nerve growth factor (NGF) (obtained from Upstate Biotechnology, Lake Placid, NY) or 25 ng/ml NGF and 5% TSTIM (a culture supplement prepared from rats and containing concanavalin A which was obtained from BD Biosciences). In addition, the following additives were added to the cells: water (vehicle control); 5 gg/mL of the compound of formula II (Cpd II); 15 µg/ml Cpd II; or 30 µg/ml of Cpd II. After 72 hours of culture at 37 °C and 5% CO₂, cell proliferation was evaluated by the Promega cell titer assay as described in Example 1. The results are shown in Table 3 below.

**TABLE 3**

| **Additive** | **Mean OD 530 nm** |
|---|---|
| No additives | 0.058 ± 0.008 |
| NGF | 0.116 ± 0.029 |
| NGF + water | 0.101 ± 0.022 |
| NGF + 1 µg/ml Cpd II | 0.117 ± 0.015 |
| NGF + 5 µg/ml Cpd II | 0.108 ± 0.012 |
| NGF + 15 µg/ml Cpd II | 0.049 ± 0.016 |
| NGF + TSTIM | 0.490 ± 0.047 |
| NGF + TSTIM + water | 0.365 ± 0.026 |
| NGF + TSTIM + 1 µg/ml Cpd II | 0.428 ± 0.027 |
| NGF + TSTIM + 5 µg/ml Cpd II | 0.373 ± 0.016 |
| NGF + TSTIM + 15 µg/ml Cpd II | 0.326 ± 0.024 |

### EXAMPLE 7

Passage 4 (*i.e*., four cell population doublings) human umbilical vein endothelial cells (HUVECs), human source lot number 9713 (obtained from ATCC), were put into the wells of a 48-well tissue culture plate at 20,000 cells/well in 500 µl of endothelial growth medium- 2 (EGM-2) complete medium (but without serum or ascorbate) (obtained from Cambrex) supplemented with ITSS (insulin, transferrin and sodium selenite) (obtained from Sigma). Also, passage 4 HUVECs, human source lot number 7016 (obtained from ATCC), were put into the wells of a 48-well tissue culture plate at 20,000 cells/well in 500 µl of EGM-2 complete medium (but without serum or ascorbate) supplemented with ITSS. The following additives were added to the cells: water (vehicle control) and 15 µg/ml of the compound of formula II (Cpd II). After incubation for 1 hour at 37 °C and 5% CO₂, lipopolysaccharide (LPS) (obtained from Sigma) was added to give a final concentration of 200 ng/ml, and the cells were incubated overnight at 37°C and 5% CO₂. After this incubation, the supernatants were collected, and the amount of IL-8 in the supernatants determined by ELISA as described in Example 4.

The results are shown in Table 4 below. As can be seen in Table 4, Cpd II complete eliminated IL-8 release by the 7016 HUVECs and decreased IL-8 release by 90% in the 9713 HUVECs.

**TABLE 4**

| Cells | Treatment | IL-8 (pg/ml) |
|---|---|---|
| 7016 HUVECs | Control (LPS only) | 53.3 |
| 7016 HUVECs | LPS + 15 µg/ml Cpd II | Below detection |
| 9713 HUVECs | Control (LPS only) | 485.0 |
| 9713 HUVECs | LPS + 15 µg/ml Cpd II | 49.8 |

### EXAMPLE 8

Passage 4 HUVECs, human source lot number 8710 (obtained from ATCC), were put into the wells of a 24-well tissue culture plate at 5,000 cells/well in EGM-2 medium (obtained from Cambrex) and cultured for 72 hours at 37°C and 5% CO₂. Then, the medium was replaced with fresh medium, and the following additives were added to the cells: water (vehicle control); 1 µg/ml, 5 µg/ml, 10 µg/ml, 15 µg/ml or 30 µg/ml of the compound of formula II (Cpd II); 15 µg/ml methylphenidate (MP); 10 µM LY 294002 (a PI3 kinase inhibitor obtained from Sigma); or 10 µM dexamethasone (Dex). After incubation for 1 hour at 37°C and 5% CO₂, TNFα (obtained from Pierce) was added to give a final concentration of 10 ng/ml, and the cells were incubated for an additional 18 hours at 37°C and 5% CO₂. After this incubation, the supernatants were collected, and the amount of IL-8 in the supernatants determined by ELISA as described in Example 4.

The results are shown in Table 5 below. As can be seen in Table 5, Cpd II decreased IL-8 release stimulated by TNFα in a dose-dependent manner, although there did appear to be some cell death caused by the highest dose (30 µg/ml). Dex and MP slightly decreased IL-8 release and LY 294002 significantly decreased IL-8 release.

**TABLE 5**

| Treatment | Mean IL-8 (pg/ml) | p value | % inhibition |
|---|---|---|---|
| No additives | 207.15 ± 66.17 | | |
| 30 µg/ml Cpd II | 0 | | |
| 15 µg/ml Cpd II | 400.35 | | |
| 10 ng/ml TNFα | 34695 ± 301.9 | | |
| 10 ng/ml TNFα + water | 35572 ± 967.74 | | |
| 10 ng/ml TNFα + 30 µg/ml Cpd II | 4829.8 ± 214.13 | | 86.93% |
| 10 ng/ml TNFα + 15 µg/ml Cpd II | 20817 ± 674.63 | 0.002 | 41.72% |
| 10 ng/ml TNFα + 10 µg/ml Cpd II | 22050 ± 727.27 | 0.003 | 38.24% |
| 10 ng/ml TNFα + 5 µg/ml Cpd II | 34482 ± 2127.22 | 0.124 | 3.08% |
| 10 ng/ml TNFα + 1 µg/ml Cpd II | 53657 ± 3935.18 | 0.011 | (-51.1%) |
| 10 ng/ml TNFα + 15 µg/ml MP | 30183 ± 3448.01 | 0.051 | 15.24% |
| 10 ng/ml TNFα + 10 µM LY 294002 | 9196.1 ± 150.97 | | 74.58% |
| 10 ng/ml TNFα + 10 µM Dex | 35952 ± 2197.14 | 0.072 | 6.88% |

### EXAMPLE 9

The transcription factor NFκB (nuclear factor κB) is implicated in the regulation of the expression of a wide variety of genes that code for mediators of the immune, acute phase and inflammatory responses. There are five subunits of the NFκB family in mammals: p50, p65 (RelA), c-Rel, p52 and RelB. The p50/p65 heterodimers and the p50 homodimers are the most common dimers found the NFκB signaling pathway. NFκB can be activated by a number of stimuli, including components of bacterial cell walls, such as lipopolysaccharide, or inflammatory cytokines, such as TNFα or IL-1β.

Activator protein-1 (AP-1) is a transcription factor that is activated during the cell cycle to promote cell survival, differentiation and adaptive responses. AP-1 proteins play a role in the expression of many genes involved in proliferation and cell cycle progression. For instance, cell transformation by oncogenes that function in the growth factor signal transduction pathway, such as *ras, rasF* and *mek*, results in a high increase in AP-1 component protein expression. Therefore, AP-1 regulated genes support the invasive process observed during malignancy and metastasis. AP-1 belongs to a large family of structurally related transcription factors that includes ATFI-4, c-Fos, c-Jun, c-Myc and C/EBP. AP-1 is composed of a mixture of heterodimeric complexes of proteins derived from the Fos and Jun families, including c-Fos, FosB, Fra-1, Fra-2, c-Jun, JunB and JunD. Primarily, AP-1 dimers blind to DNA on a TPA-response element (TRE). AP-1 expression is induced by multiple stimuli such as serum, growth factors, phorbol esters, oncogenes, cytokines of the TGF-β, TNF and interferon families, neuronal depolarization and cellular stress.

Passage 5 HUVECs, human source lot number 8750 (obtained from ATCC), were grown to confluence in 25 cm² flasks in EGM-2 medium. The following additives were added to the flasks (total volume of 5 ml/flask) in EGM-2 medium containing 2% FCS, GA1000 (gentamycin), heparin and ascorbic acid (all from Cambrex): 1 µg/ml of the compound of formula II (Cpd II); 5 µg/ml Cpd II; 15 µg/ml of Cpd II; 15 µg/ml methylphenidate (MP); or 10 µM LY 294002. The flasks were incubated overnight at 37 °C, 5% CO_{2.} After this incubation, vascular endothelial growth factor (VEGF) (obtained from Sigma) was added to give a final concentration 10 ng/ml, and the flasks were incubated for an additional 30 minutes.

Then, the amount of NFκB was determined using a TransAM™ NFκB p65/NFκB p50 Transcription Factor Assay Kit and a Nuclear Extract Kit from Active Motif North America, Carlsbad, CA, according to the manufacturer's instructions. Briefly, a nuclear extract of the cells was prepared using the Nuclear Extract Kit. Then, the nuclear extract was added to the wells of the 96-well plate of the TransAM™ kit. Oligonucleotide containing an NFκB consensus binding site was immobilized in the wells, and the activated NFκB contained in the nuclear extract was bound to the oligonucleotide. Then, an antibody directed against the NFκB p65 or p50 subunit was added, and the NFκB complex bound to the oligonucleotide was detected. A secondary antibody conjugated to horseradish peroxidase (HRP) was next added to provide a colorimetric readout that was quantified by spectrophotometry (measurement at 450 nm).

The amount of c-Jun was determined using a TransAM™ AP-1 Family Transcription Factor Assay Kit and a Nuclear Extract Kit from Active Motif North America, Carlsbad, CA, according to manufacturer's instructions. Briefly, a nuclear extract of the cells was prepared using the Nuclear Extract Kit. Then, the nuclear extract was added to the wells of a 96-well plate in which oligonucleotide containing a TPA-responsive element (TRE) was immobilized. Activator protein-1 (AP-1) dimers contained in the nuclear extract were bound to this oligonucleotide and were detected using an antibody specific for c-Jun. A secondary antibody conjugated to horseradish peroxidase (HRP) was next added to provide a colorimetric readout that was quantified by spectrophotometry (measurement at 450 nm).

The results are shown in Tables 6 and 7 below. As can be seen from Table 6, VEGF treatment of HUVECs caused almost a doubling of activated NFκB as detected by the TransAM assay. Cpd II at 15 µg/ml and 5 µg/ml reduced the amount of activated NFκB back to basal levels. As can be seen from Table 7, VEGF treatment of HUVECs caused an increase of c-Jun. Cpd II at 15 µg/ml and 5 µg/ml completely eliminated the increase in the amount of c-Jun.

**TABLE 6**

| Sample | Mean OD 450 nm (NFκB) |
|---|---|
| Control (no additives) | 0.070 ± 0.002 |
| VEGF only | 0.111 ± 0.007 |
| VEGF + 15 µg/ml Cpd II | 0.060 ± 0.008 |
| VEGF + 5 µg/ml Cpd II | 0.065 ± 0.010 |
| VEGF + 1 µg/ml Cpd II | 0.097 ± 0.013 |
| VEGF + 15 µg/ml MP | 0.093 ± 0.011 |
| VEGF + 10 µM LY 294002 | 0.138 ± 0.008 |

**TABLE 7**

| Sample | Mean OD 450 nm (c-Jun) |
|---|---|
| Control (no additives) | 0.204 ± 0.016 |
| VEGF only | 0.261 ± 0.013 |
| VEGF + 15 µg/ml Cpd II | 0.204 ± 0.010 |
| VEGF + 5 µg/ml Cpd II | 0.185 ± 0.025 |
| VEGF + 1 µg/ml Cpd II | 0.221 ± 0.008 |
| VEGF + 15 µg/ml MP | 0.230 ± 0.016 |
| VEGF + 10 µM LY 294002 | 0.340 ± 0.020 |

### EXAMPLE 10

Passage 8 (human iliac artery endothelial cells (HIAECs) (obtained from ATCC; catalog no. CC-2545) were grown to confluence in 25 cm² flasks in EGM-2 medium. Eighteen hours prior to the experiment, the medium was replaced with EGM-2 medium containing 0.1% FCS plus heparin, GA1000 (gentamycin) and bovine pituitary extract (all from Cambrex) to place the cells in a resting state. To perform the experiment the medium was aspirated from the flasks, and the following additives were added to the flasks in fresh medium (total volume of 5 ml/flask): 15 µg/ml of the compound of formula II (Cpd II) or 10 µM LY 294002. The flasks were incubated 2 hours at 37°C, 5% CO_{2.} After this incubation, VEGF or TNFα was added to give a final concentration 10 ng/ml, and the flasks were incubated for an additional 30 minutes. Then, the amount of NFκB was determined using a TransAM™ NFκB p65/NFκB p50 Transcription Factor Assay Kit and a Nuclear Extract Kit from Active Motif North America, Carlsbad, CA, as described in Example 9.

The results are shown in Table 8 below. As can be seen from Table 8, TNFα treatment of HUVECs caused an extremely large increase in the amount of activated NFκB as detected by the TransAM assay. Cpd II at 15 µg/ml reduced the amount of activated NFκB about 82%. The treatment with VEGF did not result in as large an increase in activated NFκB as achieved with TNFα, but the increased amount was reduced 70% by Cpd. II.

**TABLE 8**

| Sample | Mean OD 450 nm (NFκB) | Percent Inhibition |
|---|---|---|
| | | |
| Control (no additives) | 0.174 ± 0.004 | |
| TNFα only | 0.881 ± 0.021 | |
| TNFα + 15 µg/ml Cpd II | 0.302 ± 0.003 | 81.89% |
| TNFα + 10 Mm LY 294002 | 0.810 ±0.007 | 10.04% |
| VEGF only | 0.220 ± 0.007 | |
| VEGF + 15 µg/ml Cpd II | 0.066 ± 0.005 | 70.00% |

### EXAMPLE 11

Day 18 TRiPS cells, 1x10⁶, were incubated for 30 minutes at 37°C, either with nothing added ("Nil"), with 1 µl CD3/CD28 Dynabeads (Dynal, Oslo, Norway) ("CD3/CD28 beads") per 100,000 cells, or with CD3/CD28 beads and 15 µg/ml of the compound of formula II (Cpd II). After the incubation, the cells were lysed in Cell-Lytic Mammalian Cell Extraction Reagent (Sigma). After centrifugation to pellet cellular debris, the supernatants (cell extracts) were obtained.

The cell extracts (supernatants) were then analyzed using a Custom AntibodyArray^{™} manufactured by Hypromatrix Inc., Worcester, MA, following the manufacturer's instructions. The Custom AntibodyArray^{™} is a nylon membrane blotted with antibodies to the proteins listed below. Briefly, the cell extracts were incubated with duplicate Custom AntibodyArray^{™}'s for 2 hours at room temperature with slow shaking, followed by three washes with Tris buffer (150 mM NaCl, 25 mM Tris, 0.05% Tween-20, pH 7.5). HRP-labeled antibodies specific for phosphorylated-tyrosine, phosphorylated-serine and phosphorylated-threonine in Tris buffer were added, and the arrays incubated for 2 hours. After three more washes with Tris buffer, a peroxidase-reactive luminescent substrate was added. The arrays were visualized by exposure to X-ray film. Densitometry of the X-ray films was measured by scanning and computer analysis. The results are summarized in Table 9 below.

**TABLE 9**

| **Protein** | **Effect of Cpd II on the protein in CD3/CD28 stimulated TRiPS cells** |
|---|---|
| | |
| RAP 1 | Activated |
| RAP2 | Activated |
| JAK2 | Activated |
| STAT4 | Activated |
| STAT5b | Activated |
| P13kinaseP85 | Activated |
| | |
| MEK1 | Decreased level to below basal levels (Nil control) |
| | |
| JNK1 | Decreased level back to basal levels (Nil control) |
| JNK2 | Decreased level back to basal levels (Nil control) |
| JNK3 | Decreased level back to basal levels (Nil control) |
| MEKK1 | Decreased level back to basal levels (Nil control) |
| IkB-β | Decreased level back to basal levels (Nil control) |
| IkB-r | Decreased level back to basal levels (Nil control) |
| IL-2 | Decreased level back to basal levels (Nil control) |
| IL-4 | Decreased level back to basal levels (Nil control) |
| IL-7y | Decreased level back to basal levels (Nil control) |
| | |
| 14-3-3 | Slightly decreased the level |
| STAT6 | Slightly decreased the level |
| IkB-ε | Slightly decreased the level |
| IkB-α | Slightly decreased the level |
| | |
| VAV | No effect |
| STAT2 | No effect |

### EXAMPLE 12

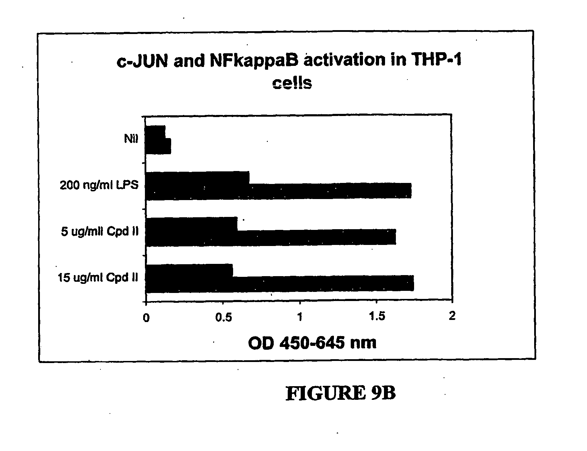

THP-1 cells were placed in medium (RPMI containing 10 % FCS and 8 ng/ml monothioglycerol) at a concentration of 250,000 cells per ml and incubated with 5 µg/ml of compound of formula II (Cpd II) or 15 µg/ml of Cpd II for one hour at 37 °C and 5% CO₂. After 1 hour, lipopolysaccharide (LPS) was added to the cultures to give a final concentration of 200 ng/ml, and the cells were then incubated for an additional 24 hours. After the incubation, the amount of NFkB and c-Jun were determined as described in Example 9. Also, the amount of c-Fos was determined using a TransAM™ AP-1 Family Transcription Factor Assay Kit and a Nuclear Extract Kit from Active Motif North America, Carlsbad, CA, according to manufacturer's instructions. Briefly, a nuclear extract of the cells was prepared using the Nuclear Extract Kit. Then, the nuclear extract was added to the wells of a 96-well plate in which oligonucleotide containing a TPA-responsive element (TRE) was immobilized. Activator protein-1 (AP-1) dimers contained in the nuclear extract were bound to this oligonucleotide and were detected using an antibody specific for c-Fos. A secondary antibody conjugated to horseradish peroxidase (HRP) was next added to provide a colorimetric readout that was quantified by spectrophotometry (measurement at 450 nm). The results are shown in Figures 9A-B.

### EXAMPLE 13

Day 10 TRiPS cells, 1x10⁶, were incubated with 15 µg/ml of the compound of formula II (Cpd II) for 1 hour at 37°C. Then, the cells were incubated with CD3/CD28 beads (1 µl per 100,000 cells) (obtained from Dynal) for 10 minutes at 37°C. The cells were then lysed with a mild buffer (supplied with Pierce EZ-Detect activation kit described below) to produce cell extracts. Protein concentrations of the resulting extracts were determined by bicinchoninic acid (BCA) assay (Pierce) and placed on ice for immediate use.

Pulldown assays were performed using Pierce EZ-Detect activation kits according to the manufacturer's instructions utilizing GST-RAF-1-RBD and GST-RalGDS-RBD for RAS and RAP-1 respectively. Briefly, 400 µg total protein from each extract was combined with recombinant protein and glutathione resin and incubated at 4°C for one hour with gentle shaking. The resin was then washed to remove unbound protein and the activated RAS and RAP-1 proteins were removed by boiling in the presence of SDS-PAGE loading dye containing reducing agent. RAS and RAP-1 western blots were performed to visualize the proteins using antibodies supplied with the kit. Densitometry of the X-ray films was done by scanning and computer analysis.

The results are shown in Table 11. As can be seen from Table 11, incubating the TRiPS cells with Cpd II resulted in very strong inhibition of RAS protein. Stimulation of the cells with CD3/CD28 beads did not increase the amount of RAP-1 protein as expected, but Cpd II also appeared to inhibit RAP-1.

**TABLE 11**

| Treatment | Integrated Optical Density for RAS assay | Integrated Optical Density for RAP-1 assay |
|---|---|---|
| No treatment | 66.83 | 259.27 |
| CD3/CD28 beads only | 245.91 | 213.66 |
| CD3/CD28 beads + 15 µg/ml Cpd II | 84.98 | 87.26 |

## Claims

1. A compound of formula I, or a salt thereof, for use in the treatment of a disease or condition comprising inhibiting an immune response, wherein formula I is: 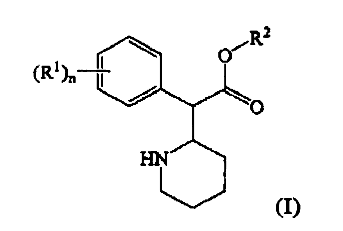 wherein
n is I;
R¹ is C₆ to C₁₄ aryl or C₆ to C₁₄ aryloxy; and
R² is methyl.

2. The compound of formula I, or a salt thereof, for use according to claim 1, wherein inhibiting an immune response comprises inhibiting the activation of T-cells.

3. The compound of formula I, or a salt thereof, for use according to claim 1 or claim 2, wherein the disease or condition is a T-cell mediated disease or condition.

4. The compound of formula I, or a salt thereof, for use according to claim 3, wherein the T-cell mediated disease or condition is an autoimmune disease or condition.

5. The compound of formula I, or a shalt thereof, for use according to claim 4, wherein the autoimmune disease or condition is multiple sclerosis.

6. The compound of formula I, or a salt thereof, for use according to claim 4, wherein the autoimmune disease or condition is systemic lupus erythematosis.

7. The compound of formula I, or a salt thereof, for use according to claim 3, wherein the T-cell mediated disease or condition is a T-cell mediated pulmonary disease.

8. The compound of formula I, or a salt thereof, for use according to claim 1, wherein inhibiting an immune response comprises inhibiting the activation of monocytes.

9. A compound of formula I, or a salt thereof, for use in the treatment of a disease or condition comprising inhibiting the production, release or both of a pro-inflammatory cytokine, wherein formula I is: 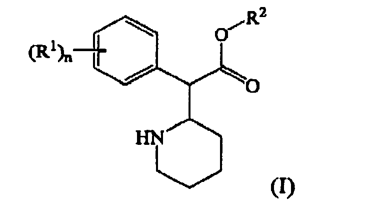 wherein
n is 1;
R¹ is C₆ to C₁₄ aryl or C₆ to C₁₄ aryloxy; and
R² is methyl.

10. The compound of formula I, or a salt thereof, for use according to claim 9, wherein the cytokine is interleukin 8 (IL-8).

11. The compound of formula I, or a salt thereof, for use according to claim 9, wherein the cytokine is interleukin 13 (IL-13).

12. The compound of formula I, or a salt thereof, for use according to claim 9, wherein the cytokine is interferon gamma (IFN-γ).

13. The compound of formula I, or a salt thereof, for use according to claim 9, wherein the cytokine is tumour necrosis factor alpha (TNF-α).

14. A compound of formula I, or a salt thereof, for use in the treatment of a disease or condition comprising inhibiting inflammation, wherein formula I is: 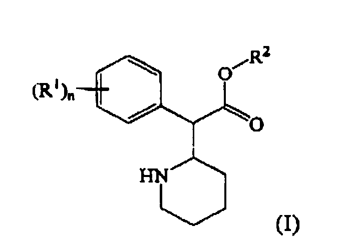 wherein
n is 1;
R¹ is C₆ to C₁₄ aryl or C₆ to C₁₄ aryloxy; and
R² is methyl.

15. The compound of formula I, or a salt thereof, for use according to claim 14, wherein the disease or condition is an inflammatory disease or condition.

16. The compound of formula I, or a salt thereof, for use according to claim 15, wherein the inflammatory disease or condition is asthma.

17. The compound of formula I, or a salt thereof, for use according to claim 15, wherein the inflammatory disease or condition is an inflammatory neurological disease or condition.

18. A compound of formula I, or a salt thereof, for use in the treatment of a disease or condition comprising inhibiting the unwanted proliferation of lymphocytes, wherein formula I is: 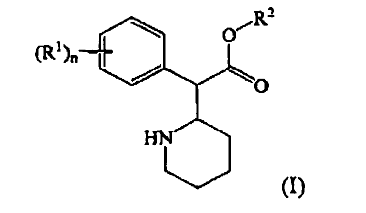 wherein n is 1;
R¹ is C₆ to C₁₄ aryl or C₆ to C₁₄ aryloxy; and
R² is methyl.

19. A compound of formula I, or a salt thereof, for use in the treatment of a disease or condition comprising inhibiting the unwanted adhesion of lymphocytes, wherein formula I is: 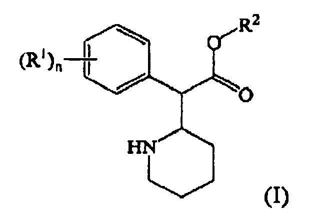 wherein
n is 1;
R¹ is C₆ to C₁₄ aryl or C₆ to C₁₄ aryloxy; and
R² is methyl.

20. A compound of formula I, or a salt thereof, for use according to any one of claims 1 to 19, wherein R¹ is phenyl or phenoxy.

21. A compound of formula I, or a salt thereof, for use according to any one of claims 1 to 20, wherein the compound is:

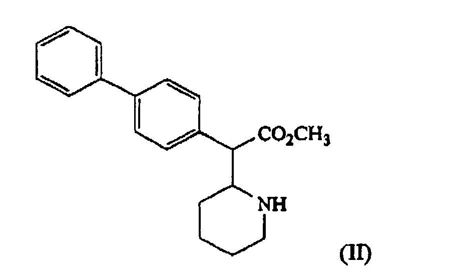

## Patentansprüche

1. Verbindung der Formel I oder ein Salz davon zur Verwendung bei der Behandlung einer Erkrankung oder einer Krankheit, die das Unterdrücken einer Immunantwort umfasst, wobei die Formel I: 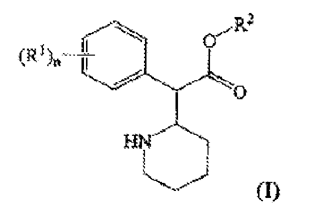 ist,
wobei
n 1 ist;
R¹ C₆- bis C₁₄-Aryl oder C₆- bis C₁₄-Aryloxy ist und
R² Methyl ist.

2. Verbindung der Formel I oder ein Salz davon zur Verwendung nach Anspruch 1, wobei das Unterdrücken einer Immunantwort das Unterdrücken der Aktivierung von T-Zellen umfasst.

3. Verbindung der Formel I oder ein Salz davon zur Verwendung nach Anspruch 1 oder 2, wobei die Erkrankung oder Krankheit eine durch T-Zellen vermittelte Erkrankung oder Krankheit ist.

4. Verbindung der Formel I oder ein Salz davon zur Verwendung nach Anspruch 3, wobei die durch T-Zellen vermittelte Erkrankung oder Krankheit eine Autoimmunerkrankung oder -krankheit ist.

5. Verbindung der Formel I oder ein Salz davon zur Verwendung na c h Anspruch 4 , wobei die Autoimmunerkrankung oder -krankheit multiple Sklerose ist.

6. Verbindung der Formel I oder ein Salz davon zur Verwendung na c h Anspruch 4 , wobei die Autoimmunerkrankung oder -krankheit systemischer Lupus erythematodes ist.

7. Verbindung der Formel I oder ein Salz davon zur Verwendung nach Anspruch 3, wobei die durch T-Zellen vermittelte Erkrankung oder Krankheit eine durch T-Zellen vermittelte Lungenerkrankung ist.

8. Verbindung der Formel I oder ein Salz davon zur Verwendung nach Anspruch 1, wobei das Unterdrücken einer Immunantwort das Unterdrücken der Aktivierung von Monozyten umfasst.

9. Verbindung der Formel I oder ein Salz davon zur Verwendung bei der Behandlung einer Erkrankung oder einer Krankheit, die das Unterdrücken der Produktion, Freisetzung oder von beidem eines proinflammatorischen Zytokins umfasst, wobei die Formel I: 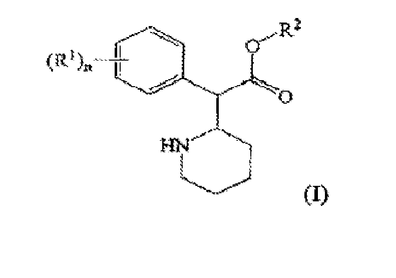 ist,
wobei
n 1 ist;
R¹ C₆- bis C₁₄-Aryl oder C₆- bis C₁₄-Aryloxy ist und
R² Methyl ist.

10. Verbindung der Formel I oder ein Salz davon zur Verwendung nach Anspruch 9, wobei das Zytokin Interleukin-8 (IL-8) ist.

11. Verbindung der Formel I oder ein Salz davon zur Verwendung nach Anspruch 9, wobei das Zytokin Interleukin-13 (IL-13) ist.

12. Verbindung der Formel I oder ein Salz davon zur Verwendung nach Anspruch 9, wobei das Zytokin Interferon-gamma (IFN-γ) ist.

13. Verbindung der Formel I oder ein Salz davon zur Verwendung nach Anspruch 9, wobei das Zytokin Tumornekrosefaktor-alpha (TNF-α) ist.

14. Verbindung der Formel I oder ein Salz davon zur Verwendung bei der Behandlung einer Erkrankung oder einer Krankheit, die das Unterdrücken einer Entzündung umfasst, wobei die Formel I: 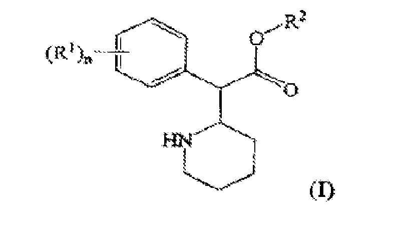 ist,
wobei
n 1 ist;
R¹ C₆- bis C₁₄-Aryl oder C₆- bis C₁₄-Aryloxy ist und
R² Methyl ist.

15. Verbindung der Formel I oder ein Salz davon zur Verwendung nach Anspruch 14, wobei die Erkrankung oder Krankheit eine entzündliche Erkrankung oder Krankheit ist.

16. Verbindung der Formel I oder ein Salz davon zur Verwendung nach Anspruch 15, wobei die entzündliche Erkrankung oder Krankheit Asthma ist.

17. Verbindung der Formel I oder ein Salz davon zur Verwendung nach Anspruch 15, wobei die entzündliche Erkrankung oder Krankheit ei n e entzündliche neurologische Erkrankung oder Krankheit ist.

18. Verbindung der Formel I oder ein Salz davon zur Verwendung bei der Behandlung einer Erkrankung oder einer Krankheit, die das Unterdrücken der unerwünschten Proliferation von Lymphozyten umfasst, wobei die Formel I: 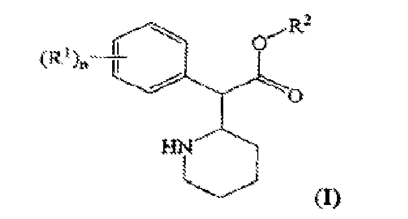 ist,
wobei
n 1 ist;
R¹ C₆- bis C₁₄-Aryl oder C₆- bis C₁₄-Aryloxy ist und
R² Methyl ist.

19. Verbindung der Formel I oder ein Salz davon zur Verwendung bei der Behandlung einer Erkrankung oder einer Krankheit, die das Unterdrücken der unerwünschten Adhäsion von Lymphozyten umfasst, wobei die Formel I: 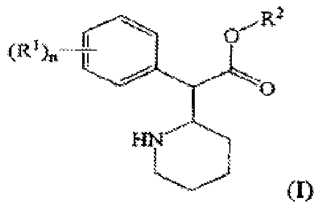 ist,
wobei
n 1 ist;
R¹ C₆- bis C₁₄-Aryl oder C₆- bis C₁₄-Aryloxy ist und
R² Methyl ist.

20. Verbindung der Formel I oder ein Salz davon zur Verwendung nach einem der Ansprüche 1 bis 19, wobei R¹ Phenyl oder Phenoxy ist.

21. Verbindung der Formel I oder ein Salz davon zur Verwendung nach einem der Ansprüche 1 bis 20, wobei die Verbindung: 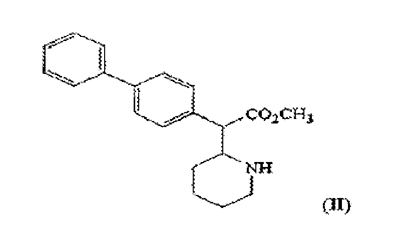 ist.

## Revendications

1. Composé de formule I, ou sel de celui-ci, destiné à être utilisé dans le traitement d'une maladie ou affection comprenant l'inhibition d'une réponse immunitaire, la formule I étant : 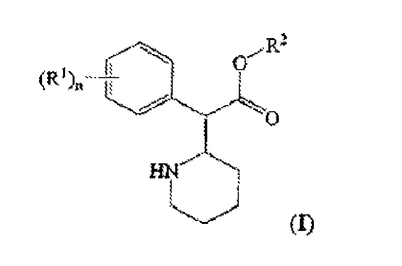 où
n vaut 1 ;
R¹ est un aryle en C₆ à C₁₄ ou un aryloxy en C₆ à C₁₄ ; et
R² est un méthyle.

2. Composé de formule I, ou sel de celui-ci, destiné à être utilisé selon la revendication 1, l'inhibition d'une réponse immunitaire comprenant l'inhibition de l'activation de lymphocytes T.

3. Composé de formule I, ou sel de celui-ci, destiné à être utilisé selon la revendication 1 ou la revendication 2, la maladie ou affection étant une maladie ou affection à médiation par les lymphocytes T.

4. Composé de formule I, ou sel de celui-ci, destiné à être utilisé selon la revendication 3, la maladie ou affection à médiation par les lymphocytes T étant une maladie ou affection auto-immune.

5. Composé de formule I, ou sel de celui-ci, destiné à être utilisé selon la revendication 4, la maladie ou affection auto-immune étant la sclérose en plaques.

6. Composé de formule I, ou sel de celui-ci, destiné à être utilisé selon la revendication 4, la maladie ou affection auto-immune étant le lupus érythémateux disséminé.

7. Composé de formule I, ou sel de celui-ci, destiné à être utilisé selon la revendication 3, la maladie ou affection à médiation par les lymphocytes T étant une maladie pulmonaire à médiation par les lymphocytes T.

8. Composé de formule I, ou sel de celui-ci, destiné à être utilisé selon la revendication 1, l'inhibition d'une réponse immunitaire comprenant l'inhibition de l'activation de monocytes.

9. Composé de formule I, ou sel de celui-ci, destiné à être utilisé dans le traitement d'une maladie ou affection comprenant l'inhibition de la production et/ou de la libération d'une cytokine pro-inflammatoire, la formule I étant : 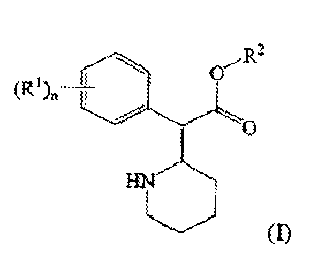 où
n vaut 1 ;
R¹ est un aryle en C₆ à C₁₄ ou un aryloxy en C₆ à C₁₄ ; et
R² est un méthyle.

10. Composé de formule I, ou sel de celui-ci, destiné à être utilisé selon la revendication 9, la cytokine étant l'interleukine 8 (IL-8).

11. Composé de formule I, ou sel de celui-ci, destiné à être utilisé selon la revendication 9, la cytokine étant l'interleukine 13 (IL-13).

12. Composé de formule I, ou sel de celui-ci, destiné à être utilisé selon la revendication 9, la cytokine étant l'interféron gamma (IFN-γ).

13. Composé de formule I, ou sel de celui-ci, destiné à être utilisé selon la revendication 9, la cytokine étant le facteur de nécrose tumorale alpha (TNF-α).

14. Composé de formule I, ou sel de celui-ci, destiné à être utilisé dans le traitement d'une maladie ou affection comprenant l'inhibition d'une inflammation, la formule I étant : 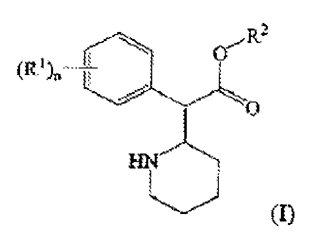 où
n vaut 1 ;
R¹ est un aryle en C₆ à C₁₄ ou un aryloxy en C₆ à C₁₄ ; et
R² est un méthyle.

15. Composé de formule I, ou sel de celui-ci, destiné à être utilisé selon la revendication 14, la maladie ou affection étant une maladie ou affection inflammatoire.

16. Composé de formule I, ou sel de celui-ci, destiné à être utilisé selon la revendication 15, la maladie ou affection inflammatoire étant l'asthme.

17. Composé de formule I, ou sel de celui-ci, destiné à être utilisé selon la revendication 15, la maladie ou affection inflammatoire étant une maladie ou affection neurologique inflammatoire.

18. Composé de formule I, ou sel de celui-ci, destiné à être utilisé dans le traitement d'une maladie ou affection comprenant l'inhibition de la prolifération non voulue de lymphocytes, la formule I étant : 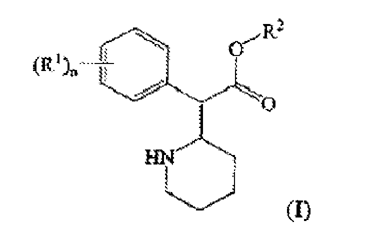 où
n vaut 1 ;
R¹ est un aryle en C₆ à C₁₄ ou un aryloxy en C₆ à C₁₄ ; et
R² est un méthyle.

19. Composé de formule I, ou sel de celui-ci, destiné à être utilisé dans le traitement d'une maladie ou affection comprenant l'inhibition de l'adhérence non voulue de lymphocytes, la formule I étant : 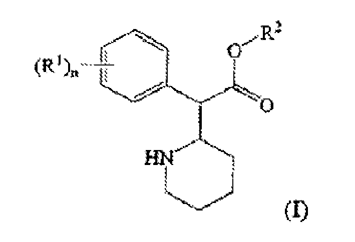 où
n vaut 1 ;
R¹ est un aryle en C₆ à C₁₄ ou un aryloxy en C₆ à C₁₄ ; et
R² est un méthyle.

20. Composé de formule I, ou sel de celui-ci, destiné à être utilisé selon l'une quelconque des revendications 1 à 19, dans lequel R¹ est un phényle ou un phénoxy.

21. Composé de formule I, ou sel de celui-ci, destiné à être utilisé selon l'une quelconque des revendications 1 à 20, le composé étant :
